(19) Europäisches Patentamt
European Patent Office
Office européen des brevets

(11) Publication number : 0 352 054 B1

# (12) EUROPEAN PATENT SPECIFICATION

(45) Date of publication of patent specification : 10.02.93 Bulletin 93/06

(51) Int. Cl.$^5$ : C09K 19/34

(21) Application number : 89307243.9

(22) Date of filing : 18.07.89

## (54) Liquid crystal compound.

(30) Priority : 19.07.88 JP 179934/88
13.06.89 JP 150795/89

(43) Date of publication of application : 24.01.90 Bulletin 90/04

(45) Publication of the grant of the patent : 10.02.93 Bulletin 93/06

(84) Designated Contracting States : CH DE FR GB LI SE

(56) References cited :
EP-A- 0 257 457
EP-A- 0 277 815
EP-A- 0 278 665
EP-A- 0 279 629
EP-A- 0 281 424
EP-A- 0 318 028

(73) Proprietor : TEIKOKU CHEMICAL INDUSTRY CO., LTD.
1-18, 1 chome, Kitahorie Nishi-ku
Osaka-shi Osaka-fu (JP)
Proprietor : SEIKO INSTRUMENTS INC.
31-1, Kameido 6-chome Koto-ku
Tokyo 136 (JP)

(72) Inventor : Ooba, Kazumasa TEIKOKU CHEMICAL IND. CO., LTD.
Itami Factory 5-41 Senzou
Itami-shi Hyougo-ken (JP)
Inventor : Suenaga, Hitoshi TEIKOKU CHEMICAL IND. CO., LTD.
Itami Factory 5-41 Senzou
Itami-shi Hyougo-ken (JP)
Inventor : Nonoguchi, Hiroshi TEIKOKU CHEMICAL IND. CO., LTD.
Itami Factory 5-41 Senzou
Itami-shi Hyougo-ken (JP)
Inventor : Taguchi, Masaaki c/o SEIKO INSTRUMENTS INC.
31-1 Kameido 6-chome Koto-ku
Tokyo (JP)
Inventor : Harada, Takamasa c/o SEIKO INSTRUMENTS INC.
31-1 Kameido 6-chome Koto-ku
Tokyo (JP)

(74) Representative : Miller, Joseph et al
J. MILLER & CO. 34 Bedford Row, Holborn
London WC1R 4JH (GB)

EP 0 352 054 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to liquid crystal compounds, and more specifically relates to phenylpyridazine compounds in which an alkyl, alkoxyalkyl, acyl or alkoxyacyl group having an asymmetric carbon atom is coupled to a benzene ring through an oxygen atom. Because of their electro-optical characteristics, these compounds may be used in various types of display devices.

One known liquid crystal compound having a phenylpyridazine structure has an alkoxy group coupled to the third position of the pyridazine ring and another alkoxy group coupled to the fourth position of the benzene ring which is connected to the sixth position of the pyridazine ring. This compound exhibits the smectic C phase (Sc phase) and may be utilised as a liquid crystal material (z., chem., vol. 17,334, 1977). Further, there has been reported another compound which has a phenylpyridazine structure where a 2-metylbutyloxy group or a 1-metylheptyloxy group is coupled to the third position of the pyridazine ring and a normal alkyl or normal alkoxy group is coupled to the fourth position of the benzene ring. Two different compounds of this type can be admixed to produce a composition exhibiting the Sc phase. The Sc phase appears at a relatively high temperature range above 50°C (Japanese Patent Publication No. 22361/1985). Another known compound having a phenylpyridazine structure has an optically active alkoxy group coupled to the third position of the pyridazine ring and a hexyloxy or octyloxy group coupled to the fourth position of the benzene ring. This type of compound does not exhibit an intermediate phase (Mol. Cryst. Liq. Cryst. Vol. 141, 25, 1986).

With these crystal compounds, the relation between the chemical structure and electro-optical characteristics is not theoretically clear. Thus, it is quite difficult to design the chemical structure of a liquid crystal compound so as to obtain desired characteristics. Under these circumstances, trial and error has been used to produce new liquid crystal compounds having better characteristics.

The present invention seeks to provide liquid crystal compounds having a ferro-electric chiral smectic phase and compounds useful as chiral dopants though not themselves having a ferro-electric chiral smectic phase.

According to one aspect of the present invention, there is provided a liquid crystal compound having a characteristic of a ferro-electric chiral smectic c phase, of the general formula:

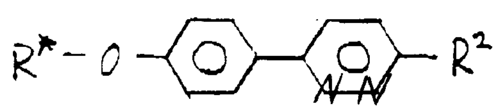

where R* is selected from an alkyl** group in which the asymetric carbon atom is situated in the $\gamma$ or $\delta$ or $\varepsilon$ or $\zeta$ (zeta) position of said alkyl group, an alk** oxyalkyl group, and an

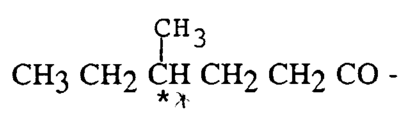

group with the mark ** indicating the position of an asymmetric carbon atom, and $R^2$ is a straight-chain alkyl group having 6 to 8 carbon atoms.

The liquid crystal compound may have a smectic C* phase.

The liquid crystal compound may have the general formula:

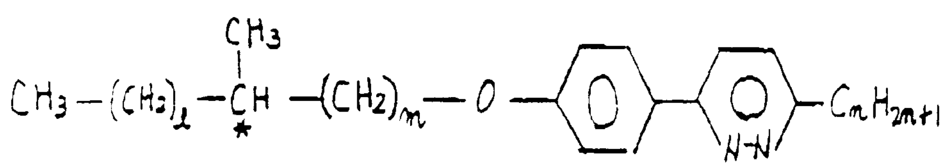

where $\ell$ is 1 to 5, m is 2 to 5, n is 6 to 8 and c* is the asymmetric carbon atom.

The liquid crystal compound may have the formula:

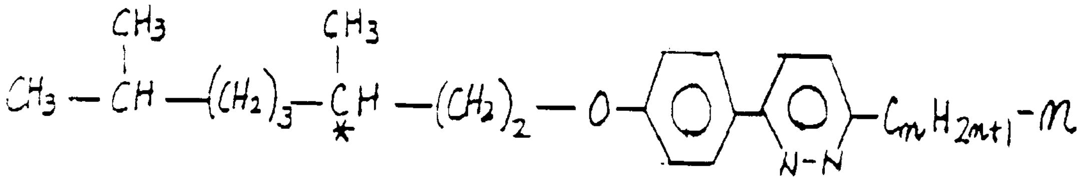

wherein c* is the asymmetric carbon atom.

The liquid crystal compound may have the general formula:

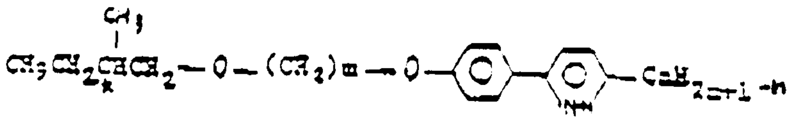

wherein m is 2-4, n is 6-8 and c* is the asymmetric carbon atom.

The liquid crystal compound may have the formula:

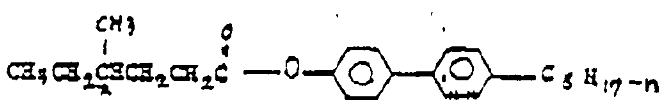

wherein c* is the asymmetric carbon atom.

According to another aspect of the present invention, there is provided a liquid crystal composition having an expanded range of transition temperature, and characterised by use of a liquid crystal compound according to the present invention.

According to a further asepct of the present invention, there is provided a liquid crystal compound according to the present invention when used as a chiral doping ingredient.

A liquid crystal compound according to the present invention may be synthesised according to the following reaction scheme:

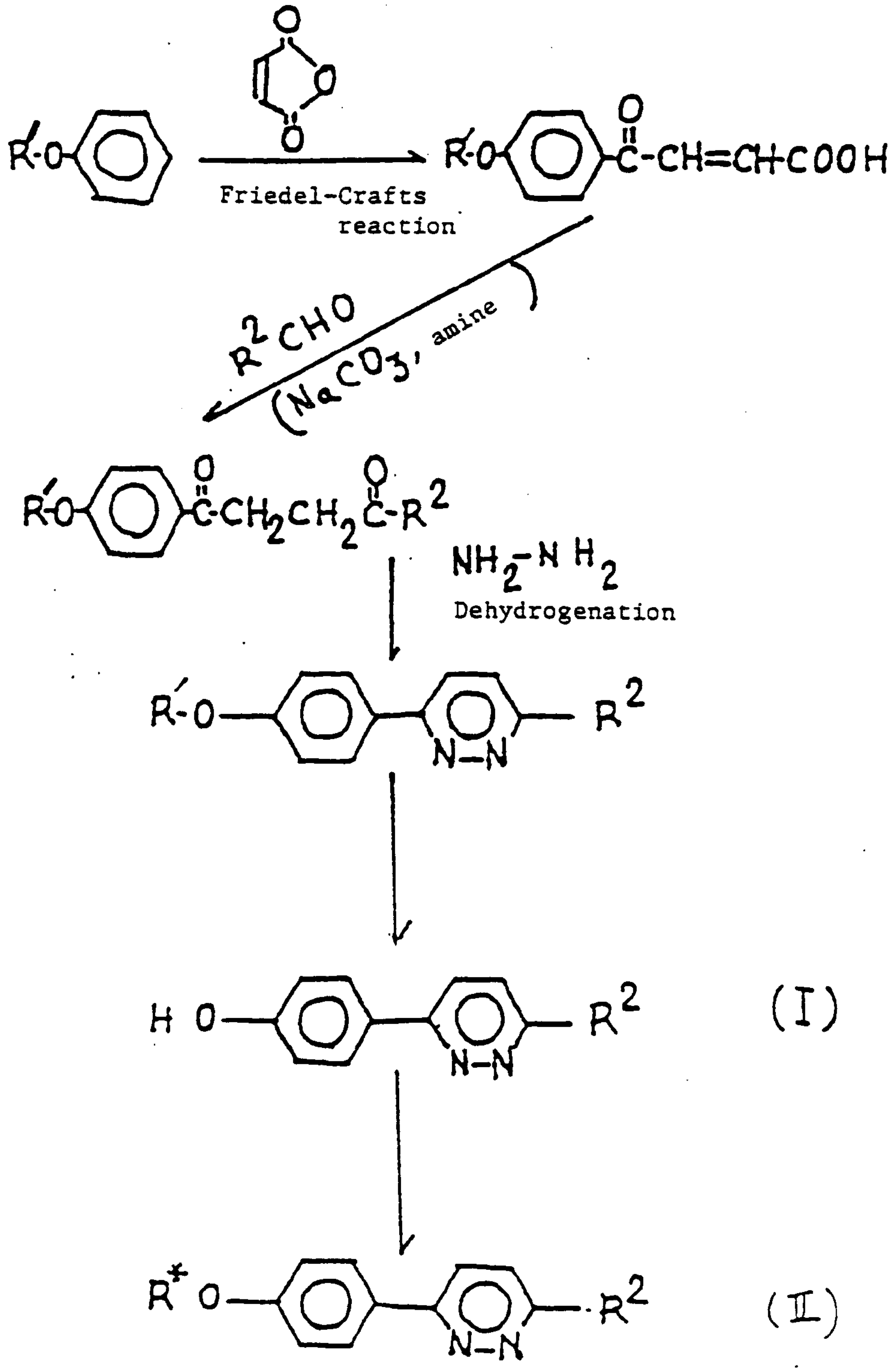


(I)

(II)

where R′ represents a group replaceable in a later reaction step and selected from a methyl, benzyl, anisyl, benzoyl and acetyl group, $R^2$ represents a straight-chain alkyl group having 6 to 8 carbon atoms and R* represents an alkyl** group in which the asymetric carbon atom is situated in the γ or δ or ε or ζ (zeta) position of said alkyl group, an alk** oxyalkyl group, or

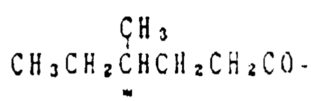

with the mark ** indicating the position of an asymmetric carbon atom.

The Friedel-Crafts reaction is conducted in a solvent such as dichloroethane trichloroethane or chloroform using a catalyst such as aluminium chloride or boron trifluoride. Next, an aliphatic straight chain aldehye, such as penthanal, hexanal, heptanal, oxanal, nonanal and decanal, is coupled. The coupling reaction is conducted in alcohol using sodium carbonate or potassium carbonate, amine and onium of 5-(2-hydroxy-ethyl)-4-methyl-1, 3-thiazoline (for example, alkylhalide and benzyl halide). Then diamine is reacted with the compound thus obtained and thereafter dehydrogenation is carried out. Then, the R′ group is removed by an appropriate method depending on the nature of the group (for example, hydrogen bromide/acetic acid reaction, hydrogen-added-dissociation and hydrolysis).

In the case of introducing an alkyl R* group into the benzene ring, the thus obtained compound represented by formula I in the reaction scheme is reacted with a halide or sulphonic acid ester or alcohol in a suitable solvent using an alkali metal agent. The alcohol is optically active and contains an asymmetric carbon atom, and is selected from 2-methylbutyl alcohol, 3-methylpentyl alcohol, 4-methylhexyl alcohol, 5-methylheptyl alcohol, 6-methyloctyl alcohol, 7-methynonyl alcohol, 8-methyldecanyl alcohol, 9-methylundecanyl alcohol, 1-methylheptyl alcohol and 2-methyloctyl alcohol. The sulphonic acid ester of these alcohols is prepared by reacting toluenesulphonylchloride or methanesulphonylchloride. The solvent is selected from dimethylformamide, dimethylsulphoxide, benzene, toluene, dichloroethane, dioxane and tetrahydrofuran. The alkali metal agent is selected from sodium hydride, sodium amide, potassium carbonate, sodium hydroxide and potassium hydroxide.

Consequently, the resulting liquid crystal compound according to the present invention and represented by formula II in the reaction scheme is obtained. The compound may or may not have a chiral smectic C phase (SmC*). If the compound does not have an SmC* phase, it may be effective to produce, in other liquid crystal material, the SmC* phase when added thereto as a dopant. Thus, the liquid crystal compound according to the present invention can be used as constituents of a ferro-electric chiral smectic liquid crystal material.

The present invention will now be described, merely by way of example, with reference to the following Examples. Examples 1 to 13 are directed to liquid crystal compounds according to the present invention having an alkyl R* group.

EXAMPLE 1

(S)-3-hexyl-6-(4-(6-methyloctyloxy) phenyl) pyridazine.

Dimethylformamide was added to 186 mg (about 60% suspension in oil) of sodium hydride and then 1.66 g of 4-(6-hexyl-3-pyridazinyl) phenol was added. The mixture was agitated for thirty minutes, and thereafter 1.93 g of tosylate of (S)-6-methyloctanol dissolved in 2 ml of dimethyl-formamide was added dropwise to the mixture. The reaction was maintained for eight hours at 80°C. After the completion of the reaction, the reaction mixture was poured into iced water and extracted using ethyl acetate. The organic layer was washed with water and dried, and thereafter condensed under reduced pressure. The product produced was purified by chromatography in a silica gel column and re-crystallised to obtain 0.8 g of a compound having the following characteristics:-

$[\alpha]^{25}_{D}$ + 4.25 (C, 2.035 chloroform)

IR spectrum $\nu^{nujol}_{cm^{-1}}$ : 1618, 1590, 1522, 1260, 835

NMR spectrum $\delta^{TMS}_{CDCl_3}$ (PPm) : 8.02, d, 2H 7.67, d, 1H 7.27, d, 1H 6.98, d, 2H 4.00, t, 2H 2.98, t, 2H 0.6 - 2.10, m, 28H

phase transition (°C) :

$$Cry \underset{77}{\overset{88}{\rightleftarrows}} SmC^{*} \xrightarrow{104} Iso$$

where, Cry denotes crystal phase, SmC* denotes chiral smectic C phase and ISO denotes isotropic liquid phase.

EXAMPLE 2

3-octyl-6-[4-(4-methylhexyloxy) phenyl] pyridazine.

In a similar manner to Example 1, using 0.85 g of 4-(6-octyl-3-pyridazinyl) phenol and 0.76 g tosylate of (S)-4-methylhexanol, there was obtained a 0.5 g of a compound having the following characteristics:-

$[\alpha]_D^{25}$ + 5.15 (c, 2.00 chloroform)

IR spectrum $\nu_{cm^{-1}}^{nujol}$ : 1618, 1586, 1522, 1258, 1035, 835

NMR spectrum $\delta_{CDCl_3}^{TMS}$ (ppm) : 8.02, d, 2H 7.69, d, 1H 7.27, d, 1H 6.99, d, 2H 4.00, t, 2H 2.98, t, 2H 0.60 - 2.16, m, 28H

phase transition (°C) :

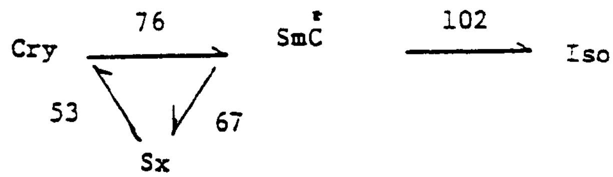


## EXAMPLES 3 TO 10

The following eight compounds were synthesised using the appropriate starting materials in similar manner to Example 1, and represented by the general formula:

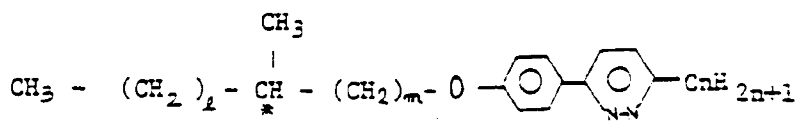

| | ℓ | m | n |
|---|---|---|---|
| Example 3 | 1 | 3 | 6 |
| Example 4 | 1 | 2 | 6 |
| Example 5 | 1 | 1 | 6 |
| Example 6 | 1 | 5 | 8 |
| Example 7 | 1 | 1 | 8 |
| Example 8 | 5 | 1 | 8 |
| Example 9 | 5 | 0 | 8 |
| Example 10 | 1 | 4 | 8 |

The transition temperatures of the resulting compounds were as follows:-

Example 3:

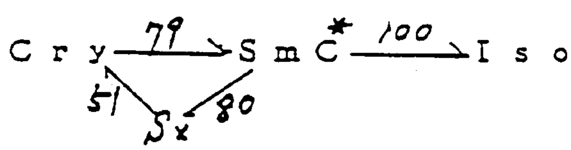


Example 4:

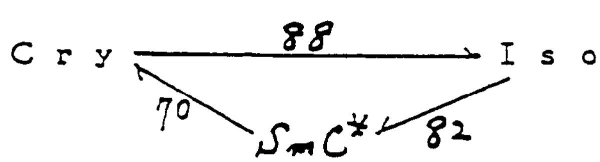


Example 5:

$$Cry \underset{88}{\overset{93}{\rightleftharpoons}} Iso$$

Example 6:

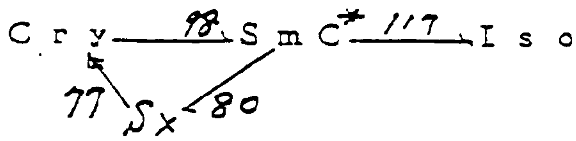


Example 7:

$$Cry \xrightarrow{93} Iso$$

Example 8:

$$Cry \xrightarrow{85} Iso$$

Example 9:

$$Cry \xrightarrow{54} Iso$$

Example 10:

$$Cry \underset{44}{\overset{63}{\rightleftharpoons}} SmC^{*} \xrightarrow{95} Iso$$

EXAMPLE 11

The compounds of each of Examples 1, 3, 7 and 9 were added to mixture A of:-

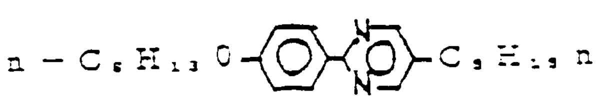

and

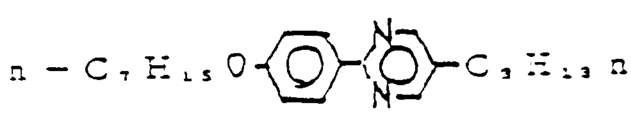

in a ratio of 1:1 by weight, the mixture A having the following phase transition mode:

$$Cry \overset{24}{\rightleftharpoons} Sc \overset{49}{\rightleftharpoons} S_A \overset{71}{\rightleftharpoons} Iso$$

where Sc denotes smectic C phase and $S_A$ denotes smectic A phase.

The liquid crystal compositions thus obtained had the following phase transition:

Mixture A : EXAMPLE 1 compound ( 1 0 : 1 w t )

Cry $\xrightarrow{21}$ SmC* $\xrightarrow{63}$ SA $\xrightarrow{70}$ Iso

Mixture A : EXAMPLE 3 compound ( 1 0 : 1 w t )

Cry $\xrightarrow{23}$ SmC* $\xrightarrow{63}$ SA $\xrightarrow{69.3}$ Ch $\xrightarrow{69.8}$ Iso

Mixture A : EXAMPLE 7 compound ( 1 0 . 1 : 1 w t )

Cry $\xrightarrow{24}$ SmC* $\xrightarrow{61}$ SA $\xrightarrow{69}$ Iso

7.2 Sx 13

Mixture A : EXAMPLE 9 compound ( 1 0 : 1 w t )

Cry $\xrightarrow{13}$ SmC* $\xrightarrow{54}$ SA $\xrightarrow{62}$ Iso

EXAMPLE 12

(S)-3-n-octyl-6-[4′-(3″-methylpentyloxy) phenyl] pyridazine represented by the following formula:

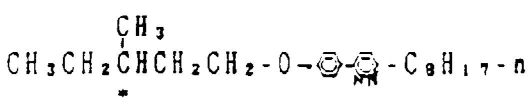

C 5 ml of dried dimethylformamide and 0.271 g of sodium hydride (about a 60% suspension in oil) were introduced into a four-way flask having a cooling tube, a dropping funnel, a temperature gauge and a calcium chloride column. Next, 1.61 g of 3-n-octyl-6-(4-hydroxyphenyl) pyridazine dissolved in 10 ml of dried dimethylformamide was added dropwise to effect reaction until generation of hydrogen gas ceased. Thereafter, 1.45 g of (S)-p-toluenesulphonic acid 3-methylpentyl ester (synthesised from active amyl alcohol) was added dropwise to effect a reaction for seven hours at 80°C. After the completion of the reaction, the resulting product was poured into iced water, and was extracted with ethyl acetate. The ethyl acetate layer was washed with water and dried, and the organic solvent was removed by evaporation under reduced pressure. The impure product obtained was refined by silica gel chromatography and re-crystallised to produce 0.9 g of (S)-n-octyl-6-[4′-(3″-methylpentyloxy) phenyl] pyridazine, having the following characteristics:-

Relative optical activity $[\alpha]_D^{25}$ + 4.85 (C=2.00 $CHC\ell_3$)

IR $\nu_{max}^{nujol}$ ($cm^{-1}$) 1618, 1522, 1260, 1180, 1120, 830

HNMR $\delta_{TMS}^{CDC\ell_3}$ (ppm)

| | | |
|---|---|---|
| 8.00 | , d | , 2H |
| 7.64 | , d | , 1H |
| 7.27 | , d | , 1H |
| 6.99 | , d | , 2H |
| 4.05 | , t | , 2H |
| 2.98 | , t | , 2H |
| 0.5 - 2.3 | , m | , 26H |

phase transition temperature (°C)

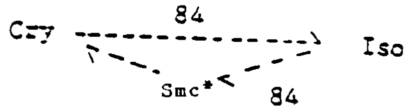


EXAMPLE 13

(R)-3-n-octyl-6-[4′-(3″,7″-dimethyloctyloxy) phenyl] pyridazine represented by the following formula:

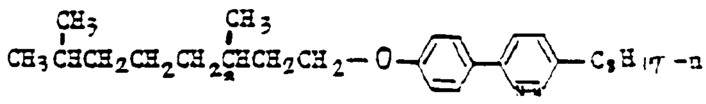

5 ml of dried dimethylformamide and 0.123 g of sodium hydride (about 60% suspension in oil) were introduced into a four-way flask having a cooling tube, a dropping funnel, a temperature gauge and a calcium chloride column. Next, 0.8 g of 3-n-octyl-6-(4-hydroxyphenyl) pyridazine dissolved in 10 ml of dried dimethylformamide was added dropwise to effect reaction until generation of hydrogen gas ceased. Thereafter, 0.881 g of (S)-p-toluenesulphonic acid 3,7-dimethyloctyl ester (synthesised from (+) - (β)-citronerol) was added dropwise to effect reaction for seven hours at 80°C. After the completion of the reaction, the resulting product was poured into iced water, and was extracted with ethyl acetate. The ethyl acetate layer was washed with water and dried, and the organic solvent was removed by evaporation under reduced pressure. The impure product obtained was refined by silica gel chromatography and re-crystallised to produce 0.7 g of (R)-n-octyl-6-[4′-(3″,7″-dimethyloctyloxy) phenyl] pyridazine having the following characteristics:

Relative optical activity $[\alpha]_D^{25}$ + 0.09 (C=2.08 ($CHC\ell_3$)

IR $\nu_{max}^{nujol}$ ($cm^{-1}$) 1620, 1525, 1420, 1260, 1180, 1120, 980, 830

HNMR $\delta_{TMS}^{CDC\ell_3}$ (ppm)

| | | |
|---|---|---|
| 7.95 | , d | , 2H |
| 7.63 | , d | , 1H |
| 7.26 | , d | , 1H |
| 6.92 | , d | , 2H |
| 3.96 | , t | , 2H |
| 2.88 | , t | , 2H |
| 0.55 - 2.15 | , m | , 34H |

phase transition temperature (°C)

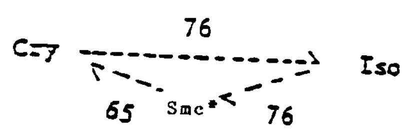


Next, various liquid crystal compounds according to the present invention having an alkoxyalkyl R* group will be described with reference to Examples 14 to 18.

EXAMPLE 14

(S)-3-n-hexyl-6-[4′-(2″-methylbutyloxypropyloxy) phenyl] pyridazine represented by the formula:-

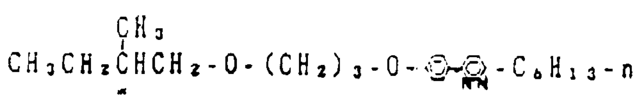

15 ml of dried N, N-dimethylformamide (hereinafter referred to as "DMF") and 0.215 g of sodium hydride (about 60% suspension in oil) were introduced into a 50 ml four-way flask provided with a cooling tube, a dropping funnel, a temperature gauge and a calcium chloride column. Next, 1.2 g of 3-n-hexyl-6-(4-hydroxyphenyl) pyridazine was added dropwise into the flask, and the reaction proceeded until generation of hydrogen gas ceased. Thereafter, 1.33 g of (S)-p-toluenesulphonic acid 2-methylbutyloxypropylester (this compound was synthesised from active amyl alcohol) was added dropwise into the flask and the reaction conducted for nine hours at 80°C. After completion of the reaction, the resulting product was poured into iced water and extracted with ether. The ether layer was washed with water and then dried, and the organic solvent was removed by evaporation under reduced pressure. The impure product obtained was refined by silica gel chromatography and re-crystallised to obtain 0.8 g of (S)-n-hexyl-6-[4′-(2″-methylbutyloxypropyloxy) phenyl] pyridazine having the following characteristics:

Relative optic activity $[\alpha]_D^{25}$ + 1.33 (C=2.03, $CHC\ell_3$)

IR $\nu_{max}^{nujol}$ ($cm^{-1}$) 1615, 1585, 1520, 1250, 1120, 835

HNMR $\delta_{TMS}^{CDC\ell_3}$ (ppm)

| | | |
|---|---|---|
| 8.06 | d | 2H |
| 7.73 | d | 1H |
| 7.31 | d | 1H |
| 7.04 | d | 2H |
| 4.15 | t | 2H |
| 3.30 | t | 2H |
| 3.28 | dd | 2H |
| 3.01 | t | 2H |
| 0.65 - 2.38 | m | 22H |

EXAMPLE 15

(S)-3-n-octyl-6-[4′-(2″-methylbutyloxypropyloxy) phenyl] pyridazine represented by the following formula:

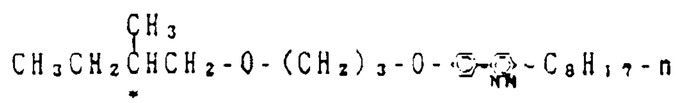

15 ml of dried DMF and 0.123 g of sodium hydride (about 60% suspension in oil) were introduced into a four-way flask having a cooling tube, a dropping funnel, a temperature gauge and a calcium chloride column. Next, 0.8 g of 3-n-octyl-6-(4-hydroxyphenyl) pyridazine dissolved in 5 ml of dried DMF was added dropwise to effect reaction until generation of hydrogen gas ceased. Thereafter, 0.8 g of (S)-p-toluenesulphonic acid 2-methylbutyloxypropyl ester (synthesised from active amyl alcohol) was added dropwise to effect reaction for ten hours at 70°C. After completion of the reaction, the resulting product was poured into iced water, and was extracted with ethyl acetate. The ethyl acetate layer was washed with water and dried, and the organic solvent was removed by evaporation under reduced pressure. The impure product obtained was refined by silica gel chromatography and re-crystallised to produce 0.6 g of (S)-n-octyl-6-[4′-(2″-methylbutyloxypropyloxy) phenyl] pyridazine, having the following characteristics:

Relative optical activity $[\alpha]_D^{25}$ + 0.96(C=2.08, $CHC\ell_3$)

IR $\nu_{max}^{nujol}$ ($cm^{-1}$) 1615, 1590, 1520, 1250, 1120, 850

HNMR $\delta_{TMS}^{CDC\ell_3}$ (ppm)

| | | |
|---|---|---|
| 7.98 | , d , | 2H |
| 7.65 | , d , | 1H |
| 7.24 | , d , | 1H |
| 6.96 | , d , | 2H |
| 4.10 | , t , | 2H |
| 3.55 | , t , | 2H |
| 3.23 | , dd, | 2H |
| 2.98 | , t , | 2H |
| 0.85 - 2.31 | , m , | 26H |

EXAMPLE 16

(S)-3-n-hexyl-6-[4′-(2″-methylbutyloxybutyloxy) phenyl] pyridazine represented by the following formula:

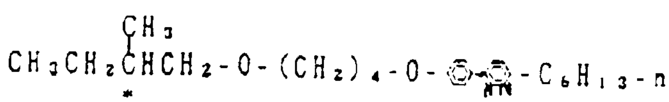

15 ml of dried DMF and 0.219 g of sodium hydride (about 60% suspension in oil) were introduced into a four-way flask having a cooling tube, a dropping funnel, a temperature gauge and a calcium chloride column. Next, 1.2 g of 3-n-hexyl-6-(4-hydroxyphenyl) pyridazine dissolved in 5 ml of dried DMF was added dropwise to effect reaction until generation of hydrogen gas ceased. Thereafter, 1.47 g of (S)-p-toluenesulphonic acid 2-methylbutyloxybutyl ester (synthesised from active amyl alcohol) was added dropwise to effect reaction for eight hours at 80°C. After the completion of the reaction, the resulting product was poured into iced water, and was extracted with ether. The ether layer was washed with water and dried, and the organic solvent was removed by evaporation under reduced pressure. The impure product obtained was refined by silica gel chromatography and re-crystallised to produce 0.85 g of (S)-n-hexyl-6-[4′-(2″-methylbutyloxybutyloxy) phenyl] pyridazine, having the following characteristics:

Relative optical activity $[\alpha]_D^{25}$ + 1.18 (C=2.025, $CHC\ell_3$)

IR $\nu_{max}^{nujol}$ ($cm^{-1}$) 1615, 1585, 1520, 1260, 1120, 830

HNMR $\delta_{TMS}^{CDC\ell_3}$ (ppm)

| | | |
|---|---|---|
| 8.07 | , d , | 2H |
| 7.75 | , d , | 1H |
| 7.33 | , d , | 1H |
| 7.05 | , d , | 2H |
| 4.07 | , t , | 2H |
| 3.49 | , t , | 2H |
| 3.28 | , dd, | 2H |
| 3.01 | , t , | 2H |
| 0.68 - 2.1 | , m , | 24H |

EXAMPLE 17

(S)-3-n-octyl-6-[4′-(2″-methylbutyloxybutyloxy) phenyl] pyridazine represented by the following formula:

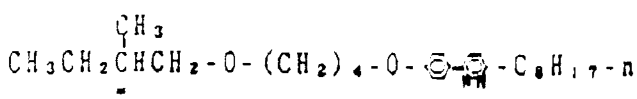

7 ml of dried DMF and 0.161 g of sodium hydride (about 60% suspension in oil) were introduced into a four-way flask having a cooling tube, a dropping funnel, a temperature gauge and a calcium chloride column. Next, 1.0 g of 3-n-octyl-6-(4-hydroxyphenyl) pyridazine dissolved in 10 ml of dried DMF was added dropwise

to effect reaction until generation of hydrogen gas ceased. Thereafter, 1.1 g of (S)-p-toluenesulphonic acid 2-methylbutyloxybutyl ester (synthesised from active amyl alcohol) was added dropwise to effection reaction for seven hours at 80°C. After completion of the reaction, the resulting product was poured into iced water, and was extracted by ethyl acetate. The ethyl acetate layer was washed with water and dried, and the organic solvent was removed by evaporation under reduced pressure. The impure product obtained was refined by silica gel chromatography and re-crystallised to produce 0.95 g of (S)-3-n-octyl-6-[4′-(2″-methylbutyloxybutyloxy) phenyl] pyridazine, having the following characteristics:

Relative optic activity $[\alpha]_D^{25}$ + 1.01 (C=2.06, $CHC\ell_3$)

IR $\nu_{max}^{nujol}$ ($cm^{-1}$) 1620, 1590, 1525, 1260, 1120, 855

HNMR $\delta_{TMS}^{CDC\ell_3}$ (ppm)

| | |
|---|---|
| 8.05 | , d , 2H |
| 7.72 | , d , 1H |
| 7.31 | , d , 1H |
| 7.07 | , d , 2H |
| 4.06 | , t , 2H |
| 3.46 | , t , 2H |
| 3.25 | , dd, 2H |
| 2.99 | , t , 2H |
| 0.67 - 2.23 | , m , 28H |

In Examples 14 to 17, the starting materials of 3-n-alkyl-6-(4-hydroxyphenyl) pyridazine and (S)-toluenesulphonic acid 2-methylbutyloxyalkyl ester are coupled to produce compounds represented by the following general formula:

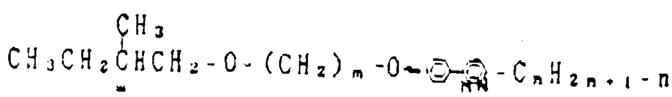

The phase transition mode of these compounds is represented as follows:

| EXAMPLE | m | n | phase transition temperature (°C) |
|---|---|---|---|
| 14 | 3 | 6 | Cry 66.5 Iso; Iso 65.5 Smc* 52 Cry |
| 15 | 3 | 8 | Cry 54 / 42 Smc* 69 Iso |
| 16 | 4 | 6 | Cry 77.5 / 63.5 Smc* 82.7 Iso |
| 17 | 4 | 8 | Cry 62 / 44 Smc* 86 Iso |

## EXAMPLE 18

Each of the compounds of Examples 14 to 17 were blended with a composition A which was a mixture of the following compounds:

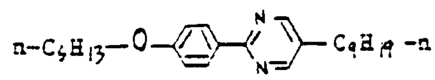

50% by weight

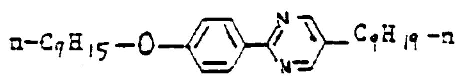

50% by weight

The phase transition mode of the resulting compositions is represented as follows:

| | mixing rate | phase transition temperature (°C) |
|---|---|---|
| Composition A | | $\mathrm{Cry} \overset{24}{\rightleftarrows} \mathrm{Sc} \overset{49}{\rightleftarrows} S_A \overset{71}{\rightleftarrows} \mathrm{Iso}$ |
| Composition A + EXAMPLE 14 | 10 : 1 | $\mathrm{Cry} \underset{12}{\rightleftarrows} \mathrm{Smc^*} \overset{60}{\rightleftarrows} S_A \overset{85}{\rightleftarrows} \mathrm{Iso}$ |
| Composition A + EXAMPLE 15 | 10 : 1 | $\mathrm{Cry} \underset{12}{\rightleftarrows} \mathrm{Smc^*} \overset{31}{\rightleftarrows} S_A \overset{67}{\rightleftarrows} \mathrm{Iso}$ |
| Composition A + EXAMPLE 16 | 10 : 1 | $\mathrm{Cry} \underset{12}{\rightleftarrows} \mathrm{Smc^*} \overset{63}{\rightleftarrows} S_A \overset{67}{\rightleftarrows} \mathrm{Iso}$ |
| Composition A + EXAMPLE 17 | 10 : | $\mathrm{Cry} \underset{11}{\rightleftarrows} \mathrm{Smc^*} \overset{63}{\rightleftarrows} S_A \overset{68}{\rightleftarrows} \mathrm{Iso}$ |

## EXAMPLE 19

This Example is directed to the synthesis of a compound according to the present invention having acyl R* group, namely, (S)-4-(6-n-octyl-3-pyridazinyl) phenyl 4′-methylhexanoic acid ester represented by the general formula:

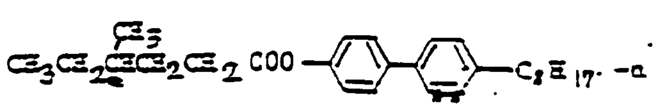

0.366 g of (S)-4-methylhexanoic acid and 0.8 g of 4-(6-n-octyl-3-pyridazinyl) phenol were dissolved in 10 ml of chloroform. Next, 0.58 g of N, N′-dicyclohexylcarbodiimide and 0.049 g of 4-dimethylaminopyridine were added to effect reaction in one day. After the completion of the reaction, insoluble products were filtered off

and the reaction products were extracted with chloroform. The organic layer was washed with 2N hydrochloric acid, a 2% solution of sodium hydroxide and water and was dried, and thereafter the remaining organic solvent was removed by evaporation under reduced pressure. The product obtained was purified by silica gel chromatography and re-crystallised to obtain 0.8 g of (S)-4-(6-n-octyl-3-pyridazinyl) phenyl 4′-methylhexanoic acid ester having the following characteristics:

Relative optical activity $[\alpha]_D^{25}$ + 5.03 (C=2.04 $CHC\ell_3$)

IR $\nu_{max}^{nujol}$ ($cm^{-1}$) 1750, 1520, 1210, 1175, 1150, 1115, 845

HNMR $\delta_{TMS}^{CDC\ell_3}$ (ppm)

| | | |
|---|---|---|
| 8.11 | , d | , 2H |
| 7.76 | , d | , 1H |
| 7.50 | , d | , 1H |
| 7.24 | , d | , 2H |
| 3.02 | , t | , 2H |
| 2.59 | , t | , 2H |
| 0.6 - 2.15 | , m | , 26H |

phase transition temperature (°C)

$$Cry \underset{41}{\overset{55}{\rightleftharpoons}} Sx \underset{70}{\overset{70}{\rightleftharpoons}} SmC^* \overset{96}{\rightleftharpoons} Iso$$

EXAMPLE 20

This Example is directed to the synthesis of a compound according to the present invention having an alkoxyacyl R* group, namely (L)-4-(6-n-octyl-3-pyridazinyl) phenyl 2′-butyloxypropionic acid ester represented by the following formula:

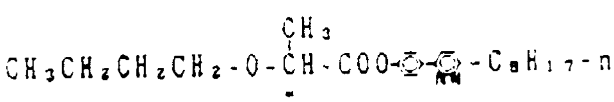

0.411 g of (L)-2-butyloxypropionic acid and 0.8 g of 4-(6-n-octyl-3-pyridazinyl) phenol were dissolved in 10 ml of dried chloroform. Next, 0.58 g of N,N′-dicyclohexylcarbodiimide and 0.034 g of 4-dimethylaminopyridine were added to effect reaction in one day. After completion of the reaction, insoluble products were filtered off and the reaction products were extracted with chloroform. The organic layer was washed with 2N hydrochloric acid, the saturated solution of sodium acid carbonate and water was dried, and thereafter the remaining organic solvent was removed by evaporation under reduced pressure. The reaction product obtained was purified by silica gel chromatography and re-crystallised to obtain 0.8 g of (L)-4-(6-n-octyl-3-pyridazinyl) phenyl 2′-butyloxypropionic acid ester having the following characteristics:

Relative optical activity $[\alpha]_D^{25}$ -38.78 (C=2.05 $CHC\ell_3$)

IR $\nu_{max}^{nujol}$ ($cm^{-1}$) 1780, 1615, 1595, 1518, 1220, 1170, 1140, 830

HNMR $\delta_{TMS}^{CDC\ell_3}$ (ppm)

| | | |
|---|---|---|
| 8.14 | , d | , 2H |
| 7.77 | , d | , 1H |
| 7.38 | , d | , 1H |
| 7.28 | , d | , 2H |
| 4.21 | , q | , 1H |
| 3.24 - 4.96 | , t | , 2H |
| 3.02 | , t | , 2H |
| 0.6 - 2.15 | , m | , 25H |

phase transition temperature (°C)

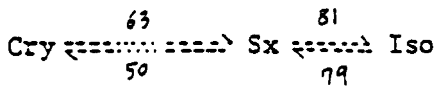


## Claims

1. A liquid crystal compound having a characteristic of a ferro-electric chiral smectic c phase, and having the general formula:

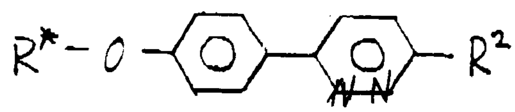

where R* is selected from an alkyl** group in which the asymetric carbon atom is situated in the γ or δ or ε or ζ (zeta) position of said alkyl group, an alk** oxyalkyl group, and an

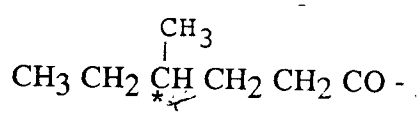

group with the mark ** indicating the position of an asymmetric carbon atom, and $R^2$ is a straight-chain alkyl group having 6 to 8 carbon atoms.

2. A liquid crystal compound as claimed in claim 1 having a smectic C* phase.

3. A liquid crystal compound as claimed in claim 1 have the general formula:

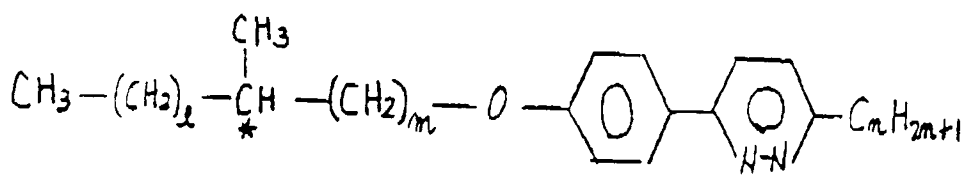

where ℓ is 1 to 5, m is 2 to 5, n is 6 to 8 and C* is the asymmetric carbon atom.

4. A liquid crystal compound as claimed in claim 1 having the formula:

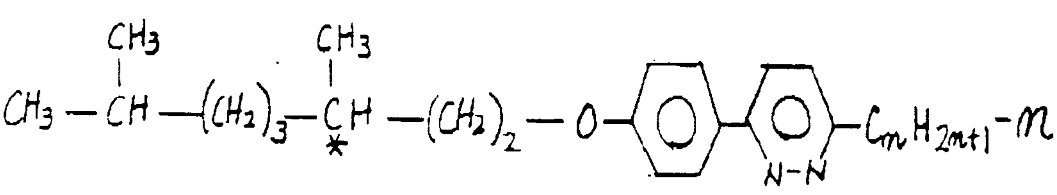

wherein C* is the asymmetric carbon atom.

5. A liquid crystal compound as claimed in claim 1 having the general formula:

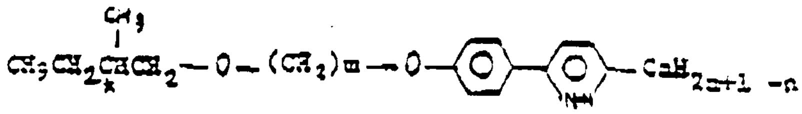

wherein m is 2-4, n is 6-8 and C* is the asymmetric carbon atom.

6. A liquid crystal compound as claimed in claim 1 having the formula:

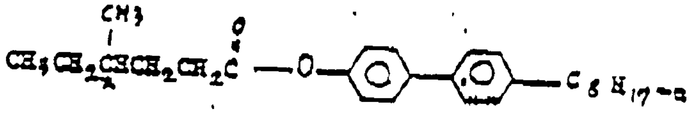

wherein C* is the asymmetric carbon atom.

7. A liquid crystal composition having an expanded range of transition temperature, characterised by use of a liquid crystal compound as claimed in claim 1.

8. A liquid crystal compound as claimed in claim 1 when used as a chiral doping ingredient.

**Patentansprüche**

1. Flüssigkristallverbindung mit einer Eigenschaft einer ferroelektrischen, chiralen, smektischen c-Phase und mit der allgemeinen Formel:

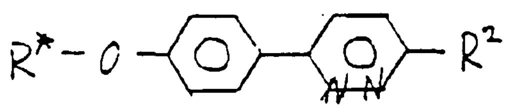

worin R* aus einer Alkyl**-Gruppe, in der sich das asymmetrische Kohlenstoffatom in der γ oder δ oder ε oder ζ (zeta) Position der Alkylgruppe befindet, einer Alk**oxyalkylgruppe und einer

$CH_3$
$CH_3\,CH_2\,CHCH_2\,CH_2\,CO$-Gruppe
*

ausgewählt ist, wobei das Zeichen ** auf die Position eines asymmetrischen Kohlenstoffatoms hinweist und $R^2$ eine geradkettige Alkylgruppe mit 6 bis 8 Kohlenstoffatomen ist.

2. Flüssigkristallverbindung nach Anspruch 1 mit einer smektischen C*-Phase.

3. Flüssigkristallverbindung nach Anspruch 1 mit der allgemeinen Formel:

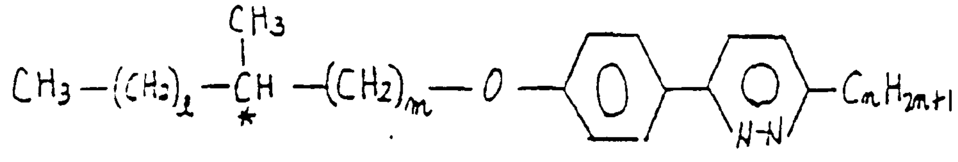

worin l 1 bis 5 ist, m 2 bis 5 ist, n 6 bis 8 ist und C* das asymmetrische Kohlenstoffatom ist.

4. Flüssigkristallverbindung nach Anspruch 1 mit der Formel:

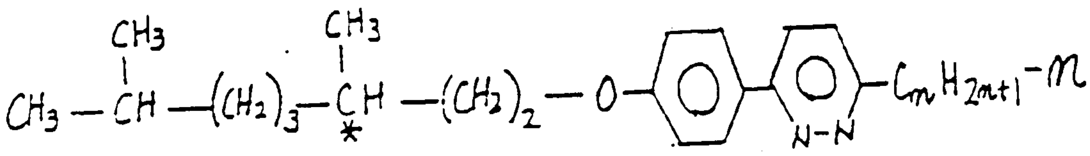

worin C* das asymmetrische Kohlenstoffatom ist.

5. Flüssigkristallverbindung nach Anspruch 1 mit der allgemeinen Formel:

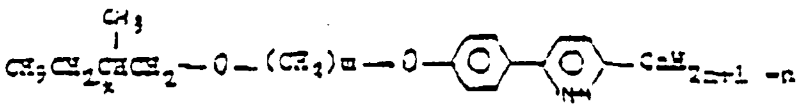

worin m 2 bis 4 ist, n 6 bis 8 ist und C* das asymmetrische Kohlenstoffatom ist.

6. Flüssigkristallverbindung nach Anspruch 1 mit der Formel:

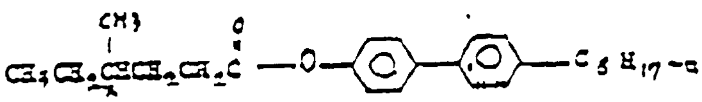

worin C* das asymmetrische Kohlenstoffatom ist.

7. Flüssigkristallzusammensetzung mit einem erweiterten Bereich der Übergangstemperatur, **gekennzeichnet durch** die Verwendung einer Flüssigkristallverbindung nach Anspruch 1.

8. Flüssigkristallverbindung nach Anspruch 1, wenn sie als chiraler Dotierungsbestandteil verwendet wird.

## Revendications

1. Un composé à cristal liquide ayant une caractéristique d'une phase smectique c chirale ferroélectrique, et ayant la formule générale :

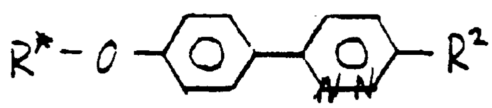

dans laquelle R* est sélectionné parmi un groupe alkyle** dans lequel l'atome de carbone asymétrique est situé dans la position $\gamma$ ou $\delta$ ou $\varepsilon$ ou $\zeta$ (zéta) au groupe alkyle, un groupe alk**oxyalkyle, et un groupe

$$CH_3\ CH_2\ \underset{*}{\overset{CH_3}{\overset{|}{CH}}}\ CH_2\ CH_2\ CO,$$

le symbole ** indiquant la position d'un atome de carbone asymétrique, et $R^2$ désignant un groupe alkyle à chaîne droite ayant de 6 à 8 atomes de carbone.

2. Un composé à cristal liquide selon la revendication, ayant une phase smectique C*.

3. Un composé à cristal liquide selon la revendication 1 ayant la formule générale :

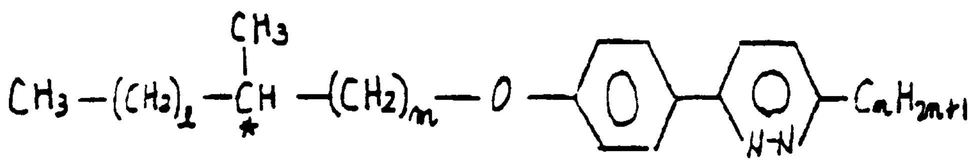

dans laquelle ℓ va de 1 à 5, m va de 2 à 5, n va de 6 à 8 et C* est l'atome de carbone asymétrique.

4. Un composé à cristal liquide selon la revendication 1 ayant la formule :

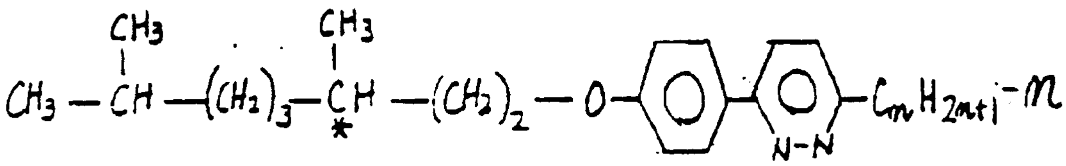

dans laquelle C* est l'atome de carbone asymétrique.

5. Un composé à cristal liquide selon la revendication 1 ayant la formule générale :

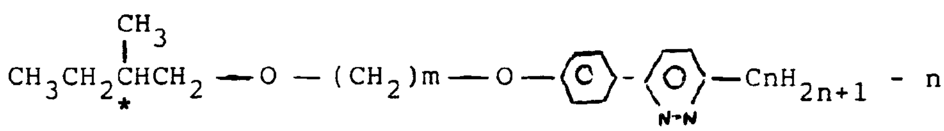

dans laquelle m va de 2 à 4, n va de 6 à 8 et C* est l'atome de carbone asymétrique.

6. Un composé à cristal liquide selon la revendication 1 ayant la formule :

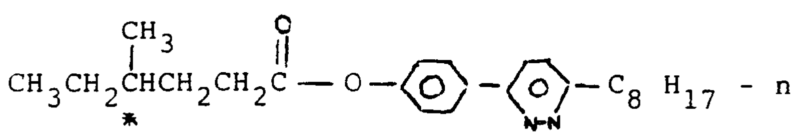

dans laquelle C* est l'atome de carbone asymétrique.

7. Une composition à cristal liquide ayant une plage étendue de température de transition, caractérisée par l'utilisation d'un composé à cristal liquide selon la revendication 1.

8. Un composé à cristal liquide selon la revendication 1, utilisé à titre d'ingrédient de dopage chiral.